# EUROPEAN PATENT APPLICATION

(11) **EP 4 112 170 A1**
(43) Date of publication of application: **04.01.2023**
(21) Application number: 21182289.5
(22) Date of filing: 29.06.2021
(51) Int. Cl.: B01L 3/00, G01N 1/40, G01N 33/68, G01N 1/28

(54) **MICROFLUIDIC DEVICES AND METHODS FOR CELL ANALYSIS**

(71) Applicant: ETH Zurich, 8092 Zürich (CH)
(72) Inventor: Cao, Xiaobao, 8049 Zürich (CH); Xue, Peng, Beijing, 100024 (CN); Aebersold, Rudolf, 8049 Zürich (CH); Stavrakis, Stavros, 8044 Zürich (CH); deMello, Andrew James, 8044 Zürich (CH)
(74) Representative: Detken, Andreas

(57) **Abstract**

In a method for analyzing cells, a sample fluid comprising a suspending medium and cells is fed to a microfluidic device comprising at least one cell processing unit (10) comprising a trapping region (12), a reaction unit (20), and an outlet arrangement (30). The trapping region is delimited by at least an input valve (13) and a sieve valve (14), in particular a v-type valve that is capable of retaining the cells while letting fluids pass. The method comprises trapping cells in the trapping region, subsequently establishing a flow of a reaction fluid through the trapping region while the sieve valve (14) assumes said open state, such that the reaction fluid transfers the trapped cells from the trapping region (12) into the reaction unit (20), decomposing the transferred cells into cell fragments through a decomposition process, collecting the cell fragments in the outlet arrangement, and analyzing the cell fragments.

## Description

### TECHNICAL FIELD

The present invention relates to methods and microfluidic devices for cell analysis, in particular single-cell proteomics.

### PRIOR ART

A major goal in biology is to provide a complete and quantitative description of cellular behavior. This task, however, has been hampered by the difficulty in assessing protein abundances and their variation with time. Unlike the genome, the human proteome is not a static entity. Protein expression levels constantly change, yielding phenotypic consequences. Conventional cellular analyses operate at the level of between 10⁵ and 10⁶ cells and thus represent ensemble measurements. Extracting data from many cells provides information on major patterns and the primary features of high abundance proteins and common signaling networks. However, averaging masks effects from cell-cycle-dependent states, inhomogeneous cellular responses and genotypic/phenotypic variability. In addition, understanding cellular characteristics and their interactions at the single cell level provides valuable information regarding the heterogeneity of living systems.

Measuring protein copy numbers, levels of phosphorylation and protein-protein interactions at the single-cell level forms the basis of single-cell proteomics. By comparing and analyzing proteomes associated with healthy and diseased individuals, one may find "disease-specific protein molecules" that can serve as molecular targets for drugs or provide molecular markers for early disease diagnosis. Furthermore, by inducing physical cell-to-cell contact between individual cells in a controlled manner, proteomics can be used to gain a better understanding of certain interaction mechanisms, such as for example the destruction of cancer cells by T-cells.

Since direct amplification of proteins is not possible and only tiny amounts of proteins are present within single cells, single-cell analysis is an immensely challenging task. For example, 0.1-0.2 ng of protein reside within a typical somatic cell, much lower than the µg-levels required when processing conventional proteomic samples via cell lysis, protein reduction, alkylation and digestion. The volumes needed for such processes are usually on the order of hundreds of microliters using traditional devices, and thus massive dilution and sample loss are unavoidable.

Traditional methods that aim to identify or quantify a limited number of proteins within a single cell use fluorescently barcoded antibodies or DNA sequences. However, due to the fact that many antibodies have low specificity for their targets, this results in inaccurate protein detection. In addition, antibody-based assays only allow for the screening of a certain number of biomarkers for which antibodies are available. Mass cytometry is an example of such a technology, in which antibodies are conjugated with rare metal tags, for profiling of up to 100 different protein targets in a single cell. The problems of cross-reactivity between antibodies and difficulties in multiplexing a large number of assays limit its application.

Mass spectrometry (MS) has recently begun to be used to detect analytes at levels that are representative of single-cell quantities. However, the preparation and processing of such small amounts of material poses a technological challenge that needs to be solved for MS to become a useful tool in single-cell proteomics.

WO 2018/085835 discloses a chip comprising an array of nanowells into which droplets containing cells are deposited, the droplets having a volume of less than 1000 nl. Microscopic imaging is used to quantify the number of cells and determine the tissue dimensions. A cocktail of reagents is added to lyse the cells, extract and denature the proteins, as well as to reduce the disulfide bonds in proteins in a single step. The proteins are then alkylated and digested using a two-step enzymatic hydrolysis. Manipulations are conducted in a humidified chamber, and a cover plate is sealed to the nanowell chip during extended incubation steps to minimize evaporation of the nanoliter droplets. Digested peptide samples in each nanowell are collected in a fused silica capillary for MS analysis.

Although the system disclosed in WO 2018/085835 allows for processing relatively small sample volumes, the number of cells deposited in each nanowell is random. Furthermore, to add reagents into the nanowells, the cover plate needs to be removed, thus the content of the nanowells is exposed to the environment, which increases the contamination risk.

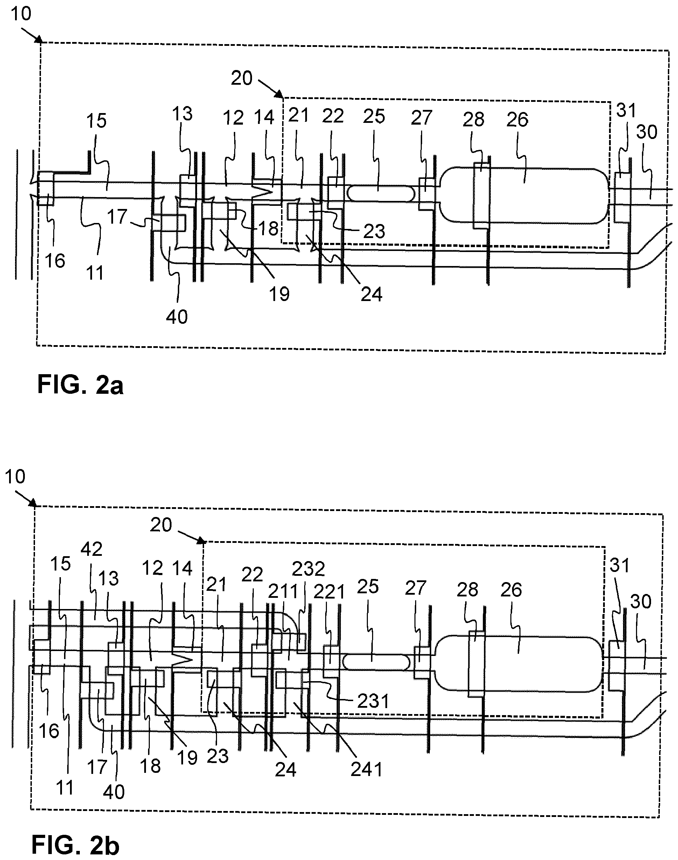

US 8877442 B2 describes a microfluidic device made of polydimethylsiloxane (PDMS) for amplifying chromosomes within a single cell. A single cell is identified under a microscope and captured from a cell suspension in a cell-sorting region A (see Fig. 2) of the microfluidic device, the region A being delimited by four 'push-up' membrane valves, the 'push-up' membrane valves being formed at locations where a control channel crosses over with a flow channel. After being captured, the cell is suspended in a protease buffer to generate a chromosome suspension in a region B. The chromosome suspension is partitioned into 48 units (region C). The content in each partition is individually amplified in an amplification region D. In the amplification region D, reagents are introduced sequentially by dead-end filling, which is possible due the gas permeability of PDMS. As the reagents are introduced, the chromosomes are pushed into one chamber after the next and finally arrive in the amplification chamber. Amplified materials are retrieved at the collection ports (region E).

With the valve arrangement used in the cell-sorting region A of the microfluidic device disclosed in US 8877442 B2, it is not possible to capture a user-determined number of cells. The cell-sorting region A is arranged at a crossing between a first channel channeling the cell suspension and a second channel, arranged perpendicularly to the first channel, channeling the protease buffer. If the valves situated in the first channel are opened to enable a new batch of cells to enter region A, any previously captured cell will inevitably be driven out of region A.

WO 2016/207320 discloses an adjustable v-type valve that can act as a sieve valve: in a first position of the adjustable valve, the valve is configured substantially not to block the fluid flow channel. Thus, in the first position of the adjustable valve, particles comprised in a fluid flowing in the fluid flow channel are not blocked by the valve and pass the valve. In a second position of the adjustable valve, the valve blocks a part of the initial passageway of the fluid flow channel. Consequently, a particle of a specific minimum size that flows with a fluid in the fluid flow channel is blocked by the adjustable valve in the second position, whereas a particle of a size smaller than that specific size is not blocked by the adjustable valve in the same second position and may pass the valve.

### SUMMARY OF THE INVENTION

In a first aspect, it is an object of the present invention to provide an improved method for analyzing cells, in particular for performing proteomic analysis on cells, the cells being protected from external contamination, which method allows the number of cells to be analyzed to be individually controlled, and which method allows individual cells to be brought into physical contact with each other in a controllable manner.

This object is achieved by a method for analyzing cells according to claim 1. Further embodiments of the invention are laid down in the dependent claims.

According to the first aspect of the invention, a method for analyzing cells is provided. The method comprises:
feeding a sample fluid comprising a suspending medium and cells suspended in the suspending medium to a microfluidic device comprising at least one cell processing unit, each cell processing unit comprising a main microchannel, the main microchannel comprising a trapping region, a reaction unit arranged downstream of the trapping region, and an outlet arrangement arranged downstream of the reaction unit, the trapping region being delimited by an arrangement of valves comprising:
   an input valve arranged at an upstream end of the trapping region, the input valve being configured for blocking and unblocking a flow of the sample fluid into the trapping region, and
   a sieve valve, in particular a v-type valve, that connects the trapping region to the reaction unit, the sieve valve being able to assume a retaining state in which the sieve valve is capable of retaining cells while letting the suspending medium pass from the trapping region into the reaction unit, and to assume an open state in which the sieve valve allows cells to pass from the trapping region into the reaction unit;
establishing a flow of the sample fluid through the trapping region by opening the input valve while the sieve valve assumes said retaining state, whereby one or more cells are trapped in the trapping region;
interrupting the flow of the sample fluid through the trapping region;
establishing a flow of a reaction fluid through the trapping region while the sieve valve assumes said open state, such that the reaction fluid transfers the trapped cells from the trapping region into the reaction unit;
decomposing the transferred cells into cell fragments through a decomposition process;
opening an outlet valve arranged between the reaction unit and the outlet arrangement, whereby the cell fragments are collected in the outlet arrangement, and analyzing the collected cell fragments.

In a second aspect, the present invention provides a microfluidic device for processing a cells, the microfluidic device comprising:
at least one cell processing unit, the at least one cell processing unit comprising a main microchannel, the main microchannel being capable of channeling a flow of a sample fluid comprising a suspending medium and cells suspended in the suspending medium, the main microchannel comprising a trapping region, a reaction unit arranged downstream of the trapping region, and an outlet arrangement arranged downstream of the reaction unit, the trapping region being delimited by an arrangement of valves comprising:
an input valve arranged at an upstream end of the trapping region, the input valve being configured for blocking and unblocking the flow of the sample fluid into the trapping region, and
a sieve valve, in particular a v-type valve, that connects the trapping region to the reaction unit, the sieve valve being able to assume a retaining state in which the sieve valve is capable of retaining cells while letting the suspending medium pass from the trapping region into the reaction unit, and to assume an open state in which the sieve valve allows cells to pass from the trapping region into the reaction unit.

The microfluidic device of the second aspect of the invention is configured to carry out the method of the first aspect of the invention.

The method may further comprise inducing cell-to-cell interactions between at least two cells trapped in the trapping region by forcing the at least two cells into physical contact in a portion of the sieve valve while the sieve valve assumes its retaining state. The at least two cells brought into physical contact are preferably cells of different cell types, such as for example cancer cells and T-cells.

To this end, the sample fluid may comprise at least two different cell types, and hence the cells trapped in the trapping region may comprise cells of at least two different cell types. Alternatively, the method may further comprise
providing at least one additional sample fluid, the at least one additional sample fluid comprising cells of a different cell type than the cell type of the cells suspended in the first sample fluid,
interrupting the flow of the first sample fluid;
establishing a flow of the at least one additional sample fluid through the trapping region, and
trapping, in the trapping region, at least one cell that has a different cell type than a previously trapped cell that is already retained in the sieve valve.

The main microchannel may comprise a selection region arranged upstream of the trapping region, the selection region being delimited by at least an entrance valve, the input valve and a rejection valve, the rejection valve connecting the selection region to a waste channel. Such a selection region may enable the following steps:
capturing at least one cell in the selection region;
imaging the at least one captured cell with an imaging device, in particular an optical microscope, and evaluating whether the at least one captured cell fulfils a set of predetermined criteria;
opening the input valve and using the flow of the sample fluid to transfer the at least one cell into the trapping region if the at least one cell fulfils the set of predetermined criteria; or
opening the rejection valve and using the flow of the sample fluid to transfer the at least one cell into the waste channel if the at least one cell does not fulfil the set of predetermined criteria. Such a cell selection process may be repeated as often as desired.

Additionally, the rejection valve may also be opened to get rid of contaminants present in the sample fluid or to rinse out any cell left over from a previous processing run in a part of the microfluidic device arranged upstream of the selection region to avoid contamination of the new cells to be processed.

The predetermined criteria may be the cell type, the cell size, the condition of the cell (i.e. whether the cell is already partially or entirely destroyed in any manner), the type of cell proteins (fluorescently labeled proteins), cell compartments (organelles such as endoplasmic reticulum, Golgi apparatus, nucleus, mitochondria, lysosomes, endosomes, and peroxisomes) or any other criteria according to which a user may wish to select a cell for further processing.

The reaction unit may comprise a first pre-chamber, the first pre-chamber being delimited by at least the sieve valve, a first connection valve that connects the first pre-chamber to a downstream portion of the reaction unit, and a first bypass valve that connects the first pre-chamber to a first bypass channel, the first bypass channel optionally opening out into a waste channel. Such a first pre-chamber may enable establishing a flow of a flushing fluid through the trapping region while at least one cell is retained by the sieve valve in its retaining state, the flushing fluid flowing from the trapping region through the sieve valve into the pre-chamber and exiting the first pre-chamber through said first bypass valve. This enables any undesired components present in the sample fluid to be flushed or washed off at the least one cell retained by the sieve valve before the at least one cell enters the reaction unit. Getting rid of such undesired components may for instance be crucial to prevent a high background signal in a subsequent analysis step involving mass spectrometry.

The downstream portion of the reaction unit may further comprise a second pre-chamber arranged downstream of the first pre-chamber, the second pre-chamber being delimited by the first connection valve, the first connection valve connecting the first pre-chamber to the second pre-chamber, the second pre-chamber being further delimited by a second connection valve that connects the second pre-chamber to a further part of the reaction unit downstream of the second pre-chamber, a second bypass valve that connects the second pre-chamber to a second bypass channel, the second bypass channel optionally opening out into the waste channel, and a flushing valve that connects the second pre-chamber to a flushing channel. Such a second pre-chamber may enable the following steps:
opening the sieve valve and using the flow of the flushing fluid to transfer the at least two cells that have been forced into physical interaction in a portion of the sieve valve into the first pre-chamber;
opening the first connection valve and using the flow of the flushing fluid to transfer at least one of said at least two cells into the second pre-chamber, while at least one of the at least two cells remains in the first pre-chamber;
closing the first connection valve;
opening the first bypass valve and using the flow of the flushing fluid to transfer the at least one remaining cell from the first pre-chamber into the first bypass channel, or
establishing a flow of the flushing fluid through the flushing channel into the second pre-chamber by opening the flushing valve and opening the second bypass valve, and using the flow of the flushing fluid to transfer the at least one cell present in the second pre-chamber from the first second pre-chamber into the second bypass channel.

By carrying out these steps, it is possible to discard cells that have participated in the cell-to-cell interaction, but which are not meant to be kept for further processing. For example, one may be interested in studying how the interaction between a T-cell and a cancer cell impacts the composition of the cancer cell. In this case, after the interaction, the T-cell may be discarded either via the first bypass channel or via the second bypass channel, while the cancer cell may be transferred to a further part of the reaction unit downstream of the second pre-chamber for further processing and analysis.

Different methods may be applied to achieve a spatial separation of the cells after their interaction in the sieve valve, i.e. to achieve a situation in which at least one cell remains in the first pre-chamber while at least one cell reaches the second pre-chamber. One may make use of the fact that the cells are likely to move at different speeds within the flow of the flushing fluid, in particular if the cells to be separated have different sizes or different shapes. Depending on their spatial arrangement while trapped in a portion of the sieve valve, the cells may also be released into the first pre-chamber at different moments in time when opening the sieve valve.

A more precise control may be achieved by using optical tweezers to manipulate the position of the cells. Alternatively, magnetic beads may be attached to the cells such that cells to which magnetic beads have been attached may be temporarily held in place using magnetic forces after being released from the sieve valve.

The arrangement of valves delimiting the trapping region may further comprise an escape valve, the escape valve connecting the trapping region to an escape channel, the escape channel optionally opening out into a waste channel. Such an escape valve enables to discard cells before they enter the processing unit. In addition, the escape valve may be opened to increase the flow rate of the sample fluid through the trapping region when the sieve valve is partially closed and thus obstructing the flow of the sample fluid, especially in case the sample fluid features a low cell density and the process of waiting for a cell to enter the trapping region exceeds a reasonable time duration.

The trapping region, like any part of the microfluidic device, may be imaged with an imaging device. The process of evaluating whether a trapped cell fulfils a set of predetermined criteria, as described above, may be implemented for a cell trapped in the trapping region, either instead of evaluating the cell in the selection region, or in addition to evaluating the cell in the selection region. By opening the escape valve and using the flow of the sample fluid, a cell that does not fulfill the set of predetermined criteria may be transferred into the escape channel and optionally into the waste channel.

In a preferred embodiment of the invention, the escape channel and the bypass channel open out into a common waste channel. This reduces the number of outlets necessary to extract waste from the microfluidic device.

Alternatively, the escape channel and/or the bypass channel may also open out into individual channels and individual outlets, for instance in a case where the user may wish to recycle a fluid or separately recollect cells that have not been processed.

Preferably, analyzing the cell fragments comprises a proteomic analysis step using mass spectrometry. In particular, a DIA/SWATH (Data Independent Acquisition/Sequential Windowed Acquisition of All Theoretical Fragment Ion Mass Spectra) method may be employed for ultra-sensitive analysis down to the single-cell level.

In particular, decomposing the cells may comprise a lysis step for extracting the cell proteome and a digestion step for fragmenting the proteome into peptides. The reaction unit may comprises a lysis chamber and a digestion chamber, the digestion chamber being separated from the lysis chamber by at least one separation valve. The lysis step may be performed in the lysis chamber using a first reaction fluid. Subsequently, a flow of a second reaction fluid may be established through the lysis chamber into the digestion chamber to transfer the lysed cells from the lysis chamber through the separation valve into the digestion chamber while the separation valve assumes an open state. Using the second reaction fluid, a digestion step may be performed in the digestion chamber. The digestion step may comprise opening and closing a stirring valve arranged in the digestion chamber in order to accelerate the digestion step. Typically, the stirring valve is actuated at a frequency of 1 Hz.

Cell decomposition processes induced by a reaction fluid are supposed to primarily take place in the reaction unit arranged downstream of the trapping region, however, since decomposition processes may start as soon as the cells enter into contact with a reaction fluid, the decomposition process may already start in the trapping region before the cells are being pushed into the reaction unit or in any region of the microfluidic device where the cells enter into contact with a reaction fluid.

Furthermore, if forced physical contact between cells is induced, depending on the type of cells, their interaction that may lead to cell decomposition before the cells even get into contact with a reaction fluid.

Analyzing the cell fragments may also comprise a genomics and/or transcriptomics analysis step. The DNA/RNA may be captured in the reaction unit using micro/nano beads and may be further amplified before undergoing sequencing.

Preferably, the microfluidic device comprises at least two cell processing units that are arranged in parallel, said at least two cell processing units being connected by a distribution channel upstream of said at least two cell processing units, wherein at least two inlet channels are arranged upstream of the distribution channel, each inlet channel being connected to the distribution channel by an inlet valve.

By arranging cell processing units in parallel, a variety of different cell processing procedures may be run in parallel, which saves time, enables higher cell throughput and helps to obtain meaningful comparisons between different processing procedures. A certain processing procedure may be carried out in parallel on different cell types, or different processing procedures may be carried out in parallel on the same cell type. The different processing procedures may vary with respect to the type of flushing fluids and reaction fluids used, with respect to the time duration for which a cell is left in a specific part of the cell processing unit or with the type of cell-to-cell interaction that may be induced.

For example, a method using a microfluidic device according to the present invention with at least two cell processing units that are arranged in parallel may comprise the following steps:
establishing a flow of a first sample fluid comprising cells of a first cell type through at least one inlet channel into the distribution channel;
causing at least one first selected cell processing unit to receive said first sample fluid from the distribution channel;
trapping cells of the first cell type in the at least one first selected cell processing unit;
interrupting the flow of the first sample fluid;
establishing a flow of at least one additional sample fluid comprising cells of at least one additional cell type through at least one inlet channel into the distribution channel;
causing at least one second selected cell processing unit to receive said at least one additional sample fluid, the at least one second selected cell processing unit being identical to the at least one first selected cell processing unit or different therefrom;
trapping cells of the at least one additional cell type in the at least one second selected cell processing unit;
interrupting the flow of the at least one additional sample fluid;
establishing a flow of at least one reaction fluid through at least one inlet channel into the distribution channel; and
causing at least one third selected cell processing unit to receive said at least one reaction fluid, the at least one third selected cell processing unit comprising the at least one first and/or second selected cell processing units.

Additional steps as described above such as selecting the cells according to predetermined criteria using an imaging device or sensitive light detectors, flushing/washing the cells using at least one flushing fluid, or forcing cells into physical contact in a portion of the sieve valve may of course also be performed in two or more cell processing units in parallel.

When at least two processing units are arranged in parallel, valves performing the same task in each processing unit may be connected such that they may be actuated simultaneously. Alternatively, the microfluidic device comprising two or more cell processing units may be configured such that each valve in each processing unit may be actuated individually in order to enable different steps to be carried out at different times in each processing unit. Of course, the microfluidic device comprising two or more cell processing units may also be configured such that some valves are connected and thus actuatable simultaneously while others are individually actuatable.

The physical dimensions of each processing unit and its constituents may be identical. Alternatively, the width and height of the microchannel, the dimensions of the valves or the volume of the chambers in the reaction unit may differ in each processing unit to enable cells of different sizes to be processed in parallel using different reaction units.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1a: shows, in a schematic top view, a microfluidic device according to a first preferred embodiment;
- Fig. 1b: shows, in a schematic top view, a microfluidic device according to a second preferred embodiment;
- Fig. 2a: shows, in a schematic top view, a first embodiment of a cell processing unit according to an embodiment of the microfluidic device;
- Fig. 2b: shows, in a schematic top view, a second embodiment of a cell processing unit according to an embodiment of the microfluidic device;
- Fig. 3: shows, in a schematic top view, an enlarged excerpt of the microfluidic device of Fig. 1b;
- Fig. 4: shows, in a schematic top view, an enlarged excerpt of the cell processing unit of Fig. 2a;
- Fig. 5: shows a schematic sectional view of the excerpt of the cell processing unit in the sectional plane A'-A' of Fig. 4;
- Fig. 6: shows a schematic sectional view of the excerpt of the cell processing unit in the sectional plane B'-B' of Fig. 4;
- Fig. 7a: shows, in a schematic top view, a v-type valve as a first embodiment of a sieve valve;
- Fig. 7b: shows a schematic sectional view of the v-type valve in the sectional place A-A of Fig. 7a in a fully open state;
- Fig. 7c: shows a schematic sectional view of the v-type valve in the sectional place B-B of Fig. 7a in a fully open state;
- Fig. 7d: shows a schematic sectional view of the v-type valve in the sectional place C-C of Fig. 7a in a fully open state;
- Fig. 7e: shows a schematic sectional view of the v-type valve in the sectional place A-A of Fig. 7a in a retaining state;
- Fig. 7f: shows a schematic sectional view of the v-type valve in the sectional place B-B of Fig. 7a in a retaining state;
- Fig. 7g: shows a schematic sectional view of the v-type valve in the sectional place C-C of Fig. 7a in a retaining state;
- Figs. 8a-b: show, in a schematic sectional view, a non-sieve valve in fully opened and fully closed state, respectively;
- Fig. 9a: shows, in a schematic top view, a second embodiment of a sieve valve in a retaining state;
- Fig. 9b: shows a schematic sectional view of the second embodiment of a sieve valve in the sectional place D-D of Fig. 9a;
- Fig. 9c: shows a schematic sectional view of the second embodiment of a sieve valve in the sectional place E-E of Fig. 9a;
- Fig. 9d: shows a schematic sectional view of the second embodiment of a sieve valve in the sectional place F-F of Fig. 9a;
- Fig. 10a: shows, in a schematic top view, a third embodiment of a sieve valve in a retaining state;
- Fig. 10b: shows a schematic sectional view of the third embodiment of a sieve valve in the sectional place G-G of Fig. 10a;
- Fig. 10c: shows a schematic sectional view of the third embodiment of a sieve valve in the sectional place H-H of Fig. 10a;
- Figs. 11a-dshow: a method of using a v-type valve to induce cell-to-cell interaction according to the present invention;
- Figs. 12a-d: show a method of separating and discarding cells after they have been trapped in a v-type valve;
- Fig. 13: shows, in a sectional view, a valve actuation input according to the microfluidic device of Fig. 1a or Fig. 1b;
- Fig. 14: shows, in a sectional view, an inlet arrangement according to the microfluidic device of Fig. 1a or Fig. 1b;
- Fig. 15: shows, in in a sectional view, a first embodiment of an outlet arrangement according to the microfluidic device of Fig. 1a or Fig. 1b;
- Fig. 16: shows, in in a sectional view, a second embodiment of an outlet arrangement according to the microfluidic device of Fig. 1a or Fig. 1b;
- Fig. 17: shows a schematic overview of a system for analyzing cells according to the present invention.

### DESCRIPTION OF PREFERRED EMBODIMENTS

### Definitions

In the present disclosure, a "sample fluid" is a fluid that comprises a suspending medium and cells suspended in the suspending medium. A "suspending medium" may be any type of fluid that may carry cells. In particular, the suspending medium may be a growth medium in which cells have been grown.

A "reaction fluid" may be any type of fluid that may induce decomposition of cells. In particular, the reaction fluid may be a lysis buffer solution or a digestion buffer solution.

A "flushing fluid" may be any type of fluid used to flush or wash the cells. In particular, the flushing fluid may be a solution that leads to a lower background signal in a mass spectrometry measurement than the sample fluid.

The term "decomposition" refers to any type of process that leads to a fragmentation of the cell or an extraction of its constituents. In particular, the term "decomposition" comprises cell lysis, cell digestion, extraction of the genome, but also physical fragmenting via mechanical forces, as e.g. caused by another cell.

In the context of the present disclosure, the term "sieve valve" is to be understood as relating to any type of valve that can be switched between a retaining state in which the valve is capable of letting a fluid pass while not allowing cells having a size above a certain minimum size suspended in said fluid to pass the valve, and an open state in which it allows said cells to pass. In particular, the sieve valve may be formed by a pneumatically actuated membrane that separates a flow channel from a control channel, the membrane getting deflected into the flow channel when actuated by increasing the pressure of a control fluid contained in the control channel, thereby gradually reducing the volume of the flow channel without closing off the flow channel completely. Accordingly, the term "non-sieve valve" is used in the present disclosure to describe valves that are capable of being switched between an open state and a closed state, the valve closing off the flow channel completely in the closed state.

The term "v-type valve" is to be understood as relating to valve formed by a pneumatically actuated membrane that separates a flow channel located in a flow layer from a control channel located in a control layer, the membrane getting deflected into the flow channel when actuated by increasing the pressure of a control fluid contained in the control channel, wherein the membrane is attached to a v-shaped protrusion formed by material of the control layer protruding into the control channel, the tip of the v-shaped protrusion being ideally located on a center axis of the flow channel and pointing in direction of the fluid flow in the flow channel, such that the cross sectional area of the flow channel that is blocked by the deflected membrane increases from an upstream end of the v-type valve to a downstream end of the v-type valve.

The term "imaging device" refers to any type of device capable of imaging cells moving within a microfluidic device according to the present invention. The imaging device may be a microscope, in particular a bright-field, dark-field, fluorescence, confocal, Raman, super-resolution or light sheet microscope.

### Preferred embodiments

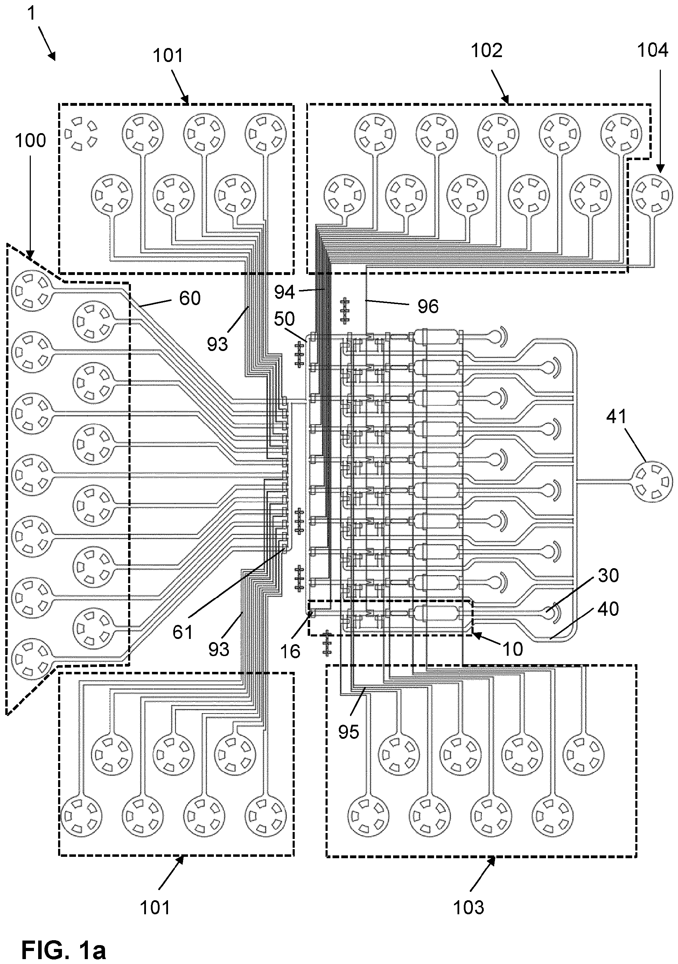

Fig. 1a illustrates, in a schematic manner, a microfluidic device 1 according to a first embodiment. The microfluidic device 1 as shown in Fig. 1a comprises ten cell processing units 10 that are arranged in parallel. The ten cell processing units 10 are connected by a distribution channel 50 arranged upstream of the cell processing units 10. Thirteen inlet channels 60 are arranged upstream of the distribution channel 50, each inlet channel 60 being connected to the distribution channel 50 by an inlet valve 61. Fluids are fed into the inlet channels 60 via inlet arrangements 100. Each inlet valve 61 is connected to an inlet valve actuation input 101 via an individual control channel 93. Each cell processing unit 10 comprises an entrance valve 16 connecting the cell processing unit to the distribution channel 50. Each entrance valve 16 is connected to an inlet valve actuation input 102 via an individual control channel 94. Each cell processing unit furthermore comprises processing valves which are non-sieve valves, in particular an input valve 13, a rejection valve 17, an escape valve 18, a connection valve 22, a bypass valve 23, a separation valve 27, a stirring valve 28 and an outlet valve 31. An outlet arrangement 30 is arranged downstream of the outlet valve 31. Each processing unit also comprises a waste channel 40. In this specific embodiment, all waste channels 40 open out into a common waste outlet 41.

In the specific embodiment shown in Fig. 1a, each type of processing valve in a certain cell processing unit 10 is connected to the processing valves of the same type of all other cell processing units via a common control channel 95, which is connected to a processing valve actuation input 103. In other words, all input valves 13 are actuated simultaneously, all rejection valves 17 are actuated simultaneously etc. (the statement is valid for all types of processing valves in the list of processing valves mentioned above). According to the present invention, each cell processing unit also comprises a sieve valve 14. In the specific embodiment shown in Fig. 1a, the sieve valves 14 of all cell processing units are connected to a common control channel 96 which common control channel 96 is in turn connected to a sieve valve actuation input 104.

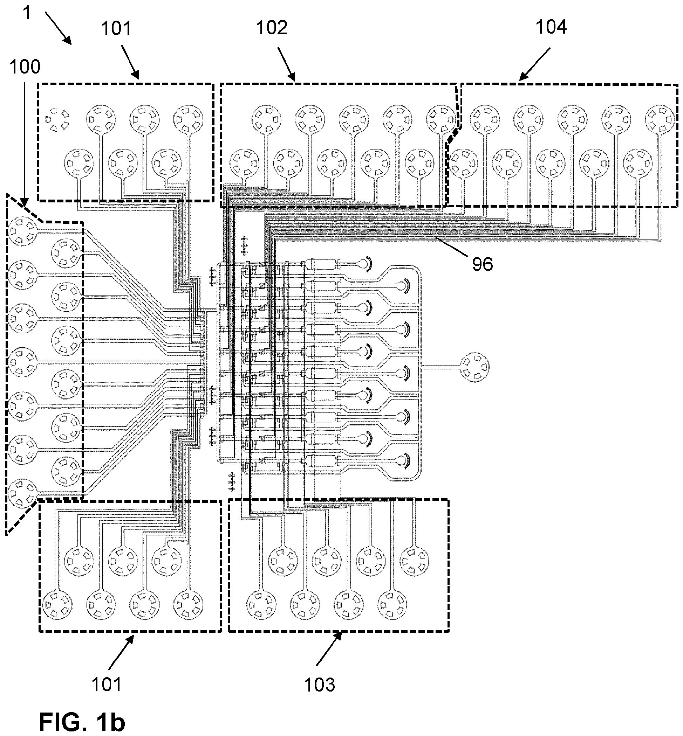

Fig. 1b illustrates, in a schematic manner, a microfluidic device 1 according to a second embodiment. The microfluidic device 1 illustrated in Fig. 1b is identical to the microfluidic device 1 illustrated in Fig. 1a except that in the second embodiment shown in Fig. 1b, the sieve valves 14 of each cell processing unit 10 are each connected to an individual control channel 96, and each individual control channel 96 is in turn connected to an individual sieve valve actuation input 104.

Fig. 2a shows an enlarged top view of a cell processing unit 10 according to a first embodiment. The cell processing unit 10 comprises a main microchannel 11, the main microchannel 11 comprising a selection region 15, a trapping region 12, a reaction unit 20, and an outlet arrangement 30. The selection region 15 is delimited by an entrance valve 16, an input valve 13 connecting the selection region to the trapping region 12, and a rejection valve 17, the rejection valve 17 connecting the selection region 15 to a waste channel 40. The trapping region 12 is delimited by the input valve 13, a sieve valve 14 connecting the trapping region 12 to the reaction unit 20 arranged downstream of the trapping region 12, and an escape valve 18. The escape valve 18 connects the trapping region 12 to an escape channel 19. The reaction unit 20 comprises a first pre-chamber 21, a lysis chamber 25 and a digestion chamber 26. The first pre-chamber 21 is delimited by the sieve valve 14, a connection valve 22 connecting the pre-chamber 21 to the lysis chamber 25, and a bypass valve 23, the bypass valve 23 connecting the pre-chamber 21 to a bypass channel 24. The digestion chamber 26 is arranged downstream of the lysis chamber 25 and is separated from the lysis chamber 25 by a separation valve 27. A stirring valve 28 is arranged in the digestion chamber 26. An outlet valve 31 is arranged downstream of the digestion chamber, connecting the digestion chamber 26 to the outlet arrangement 30. In this specific embodiment, the escape channel 19 and the bypass channel 24 both open into the waste channel 40.

Fig. 2b shows an enlarged top view of a cell processing unit 10 according to a second embodiment. This second embodiment differs from the first embodiment shown in Fig. 2a in that it further comprises a second pre-chamber 211, the second pre-chamber 211 being delimited by the first connection valve 22, the first connection valve 22 connecting the first pre-chamber 21 to the second pre-chamber 211. The second pre-chamber 211 is further delimited by a second connection valve 221 that connects the second pre-chamber 211 to the lysis chamber 25 and a second bypass valve 231 that connects the second pre-chamber 211 to a second bypass channel 241. In this specific embodiment, the second bypass channel 241 opens out into the waste channel 40. This second embodiment further comprises a flushing valve 232 that connects the second pre-chamber 211 to a flushing channel 42. The flushing channel 42 is in this case further connected to the distribution channel 50.

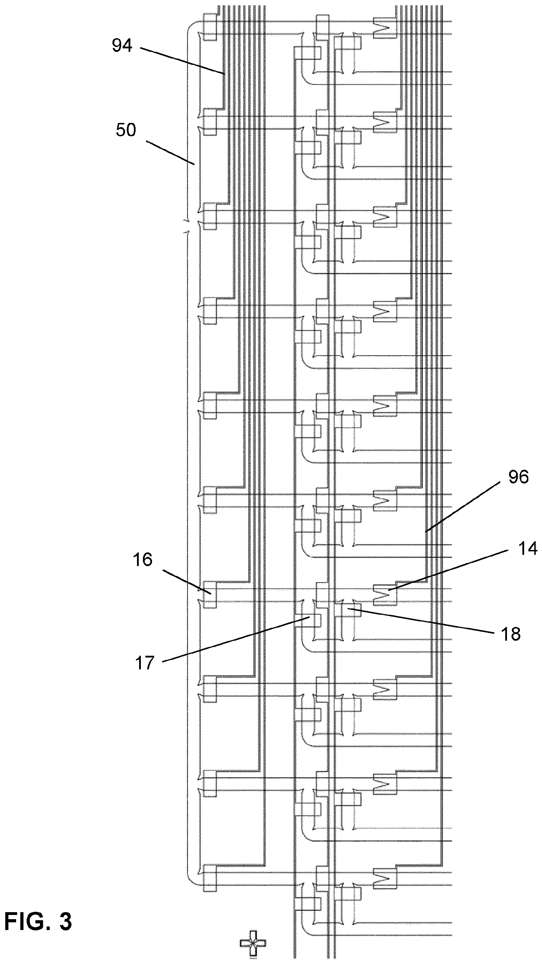

Fig. 3 shows an enlarged top view of the distribution channel 50 connecting the cell processing units 10 of the microfluidic device 1 according the second embodiment of Fig. 1b. This enlarged view shows more clearly how each sieve valve 14 is connected to its individual control channel 96.

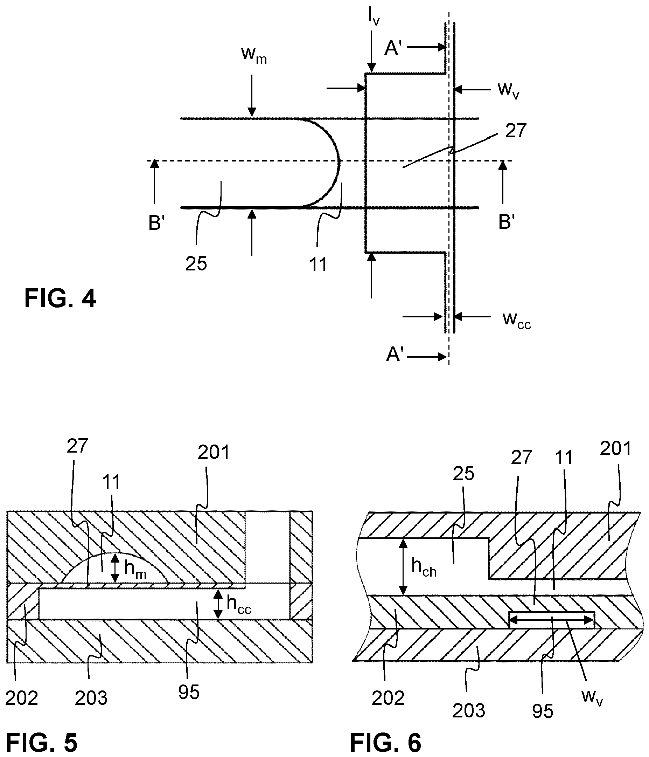

Figs. 4-6 show enlarged views of the separation valve 27 connecting the lysis chamber 25 to the digestion chamber 26 to illustrate the structure and general working principle of the valves comprised in the first and second preferred embodiment of the microfluidic device 1 shown in Fig. 1a and Fig. 1b. Fig.4-6 are to be seen as an example of a possible valve design, which may be altered without leaving the scope of the present invention.

More specifically, Fig. 4 shows a schematic top view of the separation valve 27. Fig. 5 shows a sectional view in the sectional plane A'-A' and Fig. 6 shows a sectional view of the sectional plane B'-B' of Fig. 4.

As can be seen in Fig. 5, the microfluidic device 1 comprising the separation valve 27 comprises several layers: in this specific embodiment, a flow layer 201 rests on a control layer 202, the control layer 202 in turn rests on a substrate 203. Preferably, the flow layer 201 and the control layer 202 are made of polydimethylsiloxane (PDMS), while the substrate 203 may be a glass slide.

In a first step, using fabrication processes that are well-known in the art (as for instance described by T. Thorsen et al. "Microfluidic large-scale integration", Science 298, 580-584 (2002), DOI: 10.1126/science.1076996), channels for channeling fluids are formed in the flow layer 201 and in the control layer 202. In a second step, the flow layer 201 and the control layer 202 are aligned to each other and finally brought into contact.

Here, the separation valve 27 is a membrane that is formed between the control channel 93 and the main microchannel 11 where these channels perpendicularly cross. By increasing the pressure of a control fluid 91 present in the control channel 95, the membrane will be deflected into the main microchannel 11 and thus act as a valve for fluids flowing in the main microchannel 11.

In a preferred embodiment, the cross-section of the main microchannel 11 has the shape of a segment of a circle, the segment having a width of wₘ = 100 µm and a height of hₘ= 20 µm. In the region of the lysis chamber 25, the height of the main microchannel 11 may be increased to h_{ch} = 70 µm. The control channel 95 may have a first width of w_{cc}= 10 µm and a second width of wᵥ= 100 µm where it crosses the main microchannel 11 to form the separation valve 27. Ideally, the length lᵥ (i.e. the length over which the control channel 93 is broadened to its second width wᵥ) is larger than the width wₘ of the main microchannel 11. The height of the control channel 95 may be h_{ch} = 15 µm. The volume of the lysis chamber 25 may typically be 3 nl and the volume of the digestion chamber 26 may typically be 150 nl.

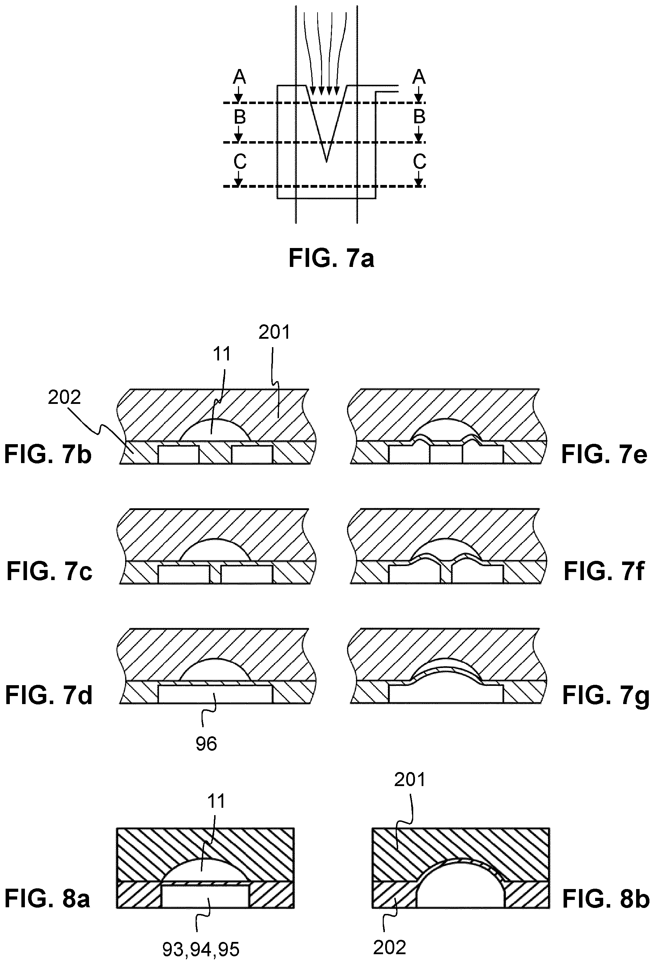

In Figs. 7a-d, an example of a v-type valve in illustrated. Fig. 7a shows a schematic top view. Figs. 7b-d show the v-type valve is in its open state in a sectional view of sectional plane A-A, B-B, and C-C marked in Fig.7a, respectively, while Figs. 7e-g show the v-type valve is in its retaining state in a sectional view of sectional plane A-A, B-B, and C-C marked in Fig.7a, respectively. As can be seen in the top view of Fig. 7a, material of the control layer 202 forms a v-shaped protrusion into an area where the control channel 96 crosses the main microchannel 11, the tip of the v-shaped protrusion being ideally located on a center axis of the main microchannel 11 and pointing in direction of the fluid flow in the main microchannel 11.

Figs. 8a-b show a non-sieve valve in fully opened and fully closed state, respectively, operating with the same principle as the separation valve 27 described in detail above.

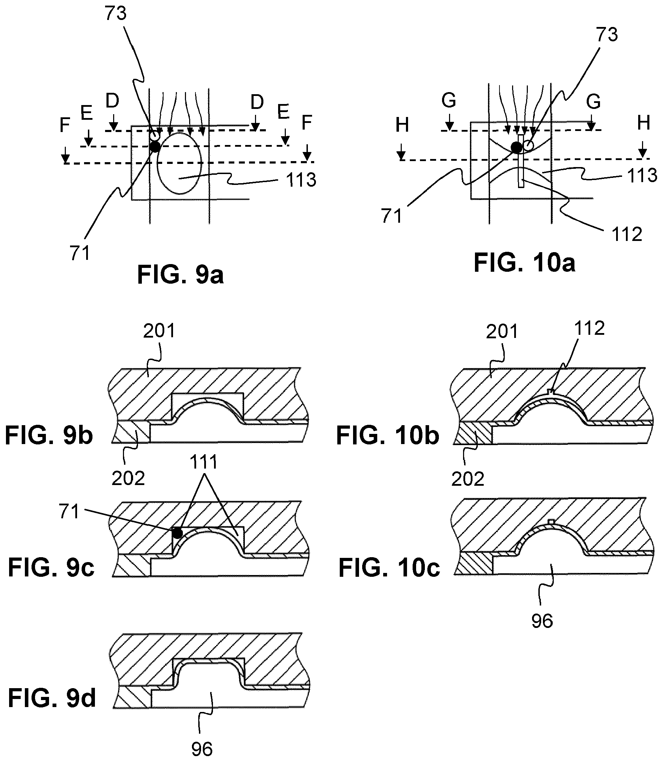

Figs. 9a-d show a second embodiment of a sieve valve. Fig. 9a shows a top view, while Figs. 9b-d show the second embodiment of a sieve valve in a retaining state in a sectional view of sectional plane D-D, E-E, and F-F marked in Fig.9a, respectively. In this second embodiment of a sieve valve, the main microchannel 11 has a rectangular cross section. The membrane therefore cannot close the main microchannel 11 completely even in its fully deflected state (the retaining state of the sieve valve). As can be seen in the top view (Fig. 9a), a contacting area 113 forms where the deflected membrane enters in contact with the flow layer. Fluids may still be able to pass around the contacting area even when the membrane is fully deflected. Depending on their size, cells 71,73 may be trapped in corner areas 111 of a sieve valve according to this second embodiment.

Figs. 10a-c show a third embodiment of a sieve valve. Fig. 10a shows a top view, while Figs. 10b and Fig. 10c show the third embodiment of a sieve valve in a retaining state in a sectional view of sectional plane G-G and H-H marked in Fig.10a, respectively. In this third embodiment of a sieve valve, the cross section of the main microchannel 11 is a circular segment that features an additional groove 112 extending radially from the circular segment further into the flow layer 201. Here, the membrane also cannot close the main microchannel 11 completely even in its fully deflected state. As can be seen in the top view (Fig. 10a), a contacting area 113 forms where the deflected membrane enters in contact with the flow layer. In this third embodiment of a sieve valve, fluids may still be able to pass through the additional groove 112 even when the valve is fully deflected. As shown in Fig. 10a, depending on their size, cells 71,73 may be trapped below the groove 112 along an upstream edge of the contacting area 113.

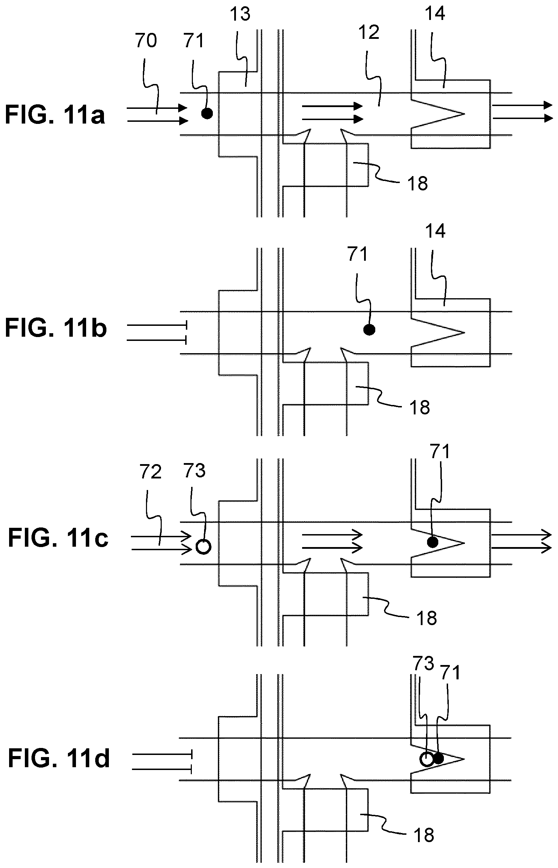

Figs. 11a-d show the trapping region 12 in a schematic top view and illustrate a method of trapping a user-determined number of cells in the trapping region 12, wherein the user-determined number of cells trapped in the trapping region 12 comprises cells of at least two different cell types.

In a first step of the method, depicted in Fig. 11a, a flow of a sample fluid 70 containing cells of a first type 71 is established through the trapping region 12 by opening the input valve 13 and putting the sieve valve 14 in a retaining state that does not allow the cells 71 to pass through the sieve valve 14. The escape valve 18 may either be open or closed during this process. Opening the escape valve 18 increases the flow rate of the sample fluid 70 through the trapping region 12 and hence reduces the time a user has to wait until at least one cell 71 has reached the trapping region 12. Closing the escape valve 18 leads to a reduced flow rate of the sample fluid 70 through the trapping region 12, since the flow is obstructed by the partially closed sieve valve 14. However, closing the escape valve 18 prevents any cell 71 from accidentally escaping the trapping region 12 through the escape valve 18. Preferably, an imaging device 2 is used to monitor the trapping region 12 and to facilitate counting of the cells 71 entering the trapping region 12. The user may opt to open or close the escape valve 18 flexibly to adjust the flow rate while monitoring the trapping region 12 through the imaging device 2 in real-time.

In a second step (Fig. 11b), after a user-determined number of cells 71 has been trapped in the trapping region, the flow of the sample fluid (70) through the trapping region (12) is interrupted, for instance by closing the input valve 13. The term "user-determined number of cells" refers in this context to any number between one and a maximum number defined by the quantity of cells that may physically fit into the trapping region 12.

In a third step (Fig. 11c), a flow of an additional sample fluid 72 comprising cells 73 of a different cell type than the cell type of the cells 71 suspended in the first sample fluid 70, is established through the trapping region 12 while keeping the sieve valve 14 in a state that does not allow the cells 71,73 to pass through the sieve valve 14. The flow of the additional sample fluid 72 may be used to push a previously trapped cell 71 further into the sieve valve 14 and subsequently also push the cell of the different cell type 73 towards the previously trapped cell 71.

In a fourth step (Fig. 11d), the flow of the additional sample fluid 72 is interrupted once the trapped cells have been brought into physical contact with each other in a portion of the sieve valve 14. The trapped cells may be left to interact with each other for a user-determined time duration.

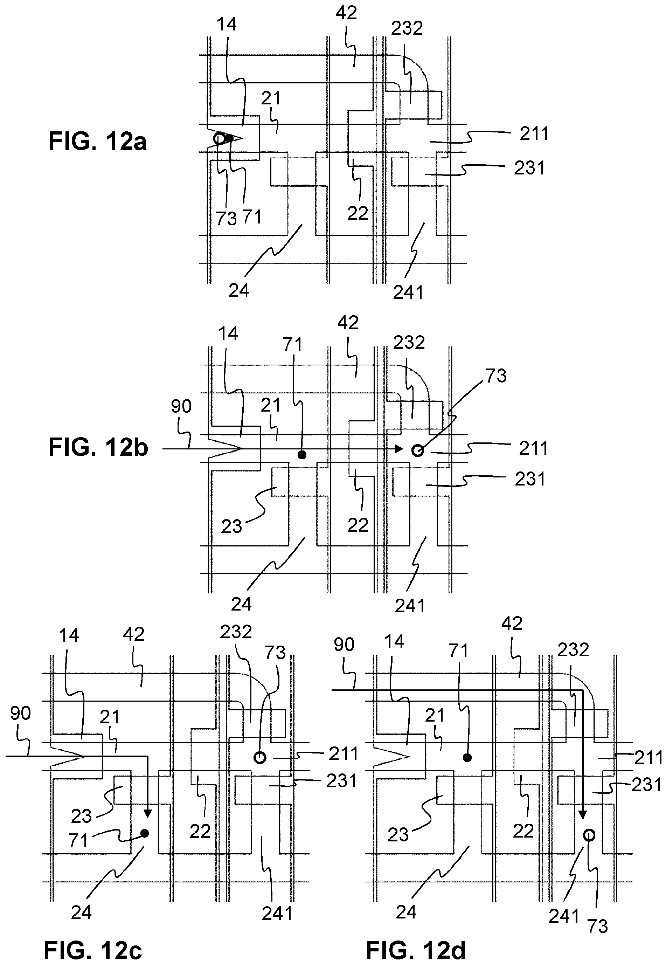

Figs. 12a-d show the first pre-chamber 21 and the second pre-chamber 211 in a schematic top view and illustrate a method of separating and discarding cells after they have been trapped in a portion of the sieve valve 14. Fig. 12a essentially corresponds to the state shown in Fig. 11d. In a next step shown in Fig. 12b, the sieve valve 14 is opened and a flow of a flushing fluid 90 is used to transfer one cell 73 into the second pre-chamber 211, while one cell remains in the first pre-chamber 21. Subsequently, the first connection valve 22 is closed and the first bypass valve 23 is opened to use the flow of the flushing fluid 90 to transfer the remaining cell 71 from the first pre-chamber 21 into the first bypass channel 24 and further into the waste channel 40 (Fig. 12c) or, alternatively, a flow of the flushing fluid 90 through the flushing channel 42 into the second pre-chamber 211 is established by opening the flushing valve 232 and opening the second bypass valve 231, and the flow of the flushing fluid 90 is used to transfer the cell 73 present in the second pre-chamber 211 from the first second pre-chamber 211 into the second bypass channel 241 and further into the waste channel 40 (Fig. 12d).

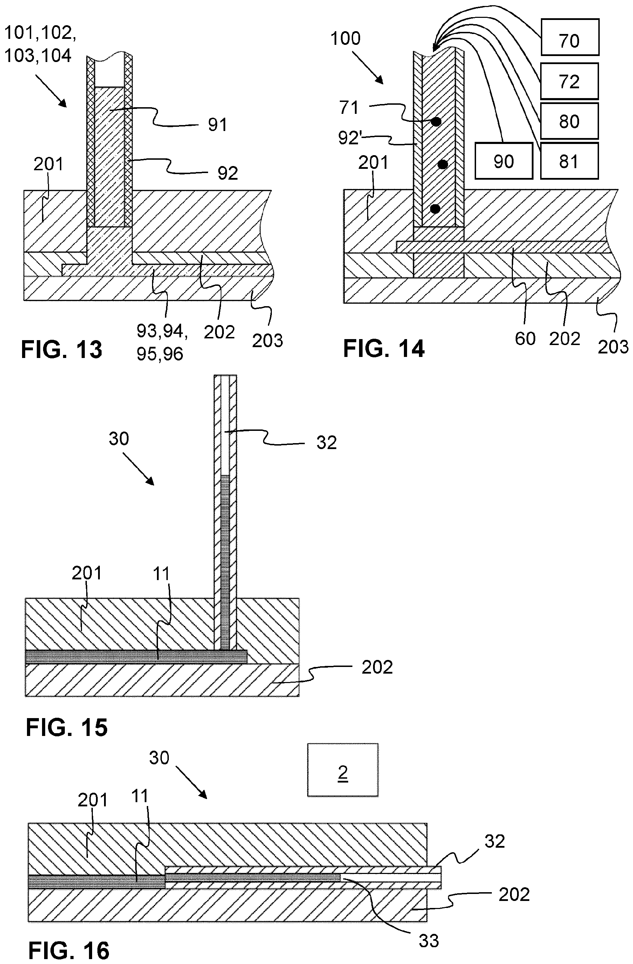

Fig. 13 illustrates a preferred embodiment of an inlet valve actuation input 101, an entrance valve actuation input 102, a processing valve actuation input 103 or a sieve valve actuation input 104. Preferably, each of these valve actuation inputs comprises a vertical opening in the flow layer 201 that is aligned to a vertical opening in the control layer 202. The aligned vertical openings open out into the control channel 93,94,95,96, located in the control layer 202. A control tube 92 is inserted into the vertical openings such that a control fluid 91, preferably water, can be filled into the control channel 93,94,95,96 via the tube 92 to actuate the valves.

Fig. 14 illustrates a preferred embodiment of an inlet arrangement 100. Preferably, the inlet arrangement 100 comprises a vertical opening in the flow layer 201. The vertical opening opens into the inlet channel 60 located in the flow layer 201. An inlet tube 92' is inserted into the vertical opening such that either a sample fluid 70,72 or a reaction fluid 80,81 or a flushing fluid 90 can be filled into the inlet channel 60 via the inlet tube 92'.

Fig.15 illustrates a first embodiment of an outlet arrangement 30, the outlet arrangement 30 comprising a capillary 32 that is inserted vertically into the flow layer 201 such that the capillary opens into the main microchannel 11 and permits a fluid containing cell fragments to be extracted from the microfluidic device 1 for further processing and analysis.

Fig.16 illustrates a second embodiment of an outlet arrangement 30, the outlet arrangement 30 comprising a capillary 32 that is inserted horizontally into the flow layer 201 such that the capillary opens into the main microchannel 11 and permits a fluid containing cell fragments to be extracted from the microfluidic device 1 for further processing and analysis. This second embodiment of an outlet arrangement 30 presents the advantage that a fluid interface 33 forming in the capillary 32 may easily be monitored using an imaging device 2 placed above the microfluidic device 1.

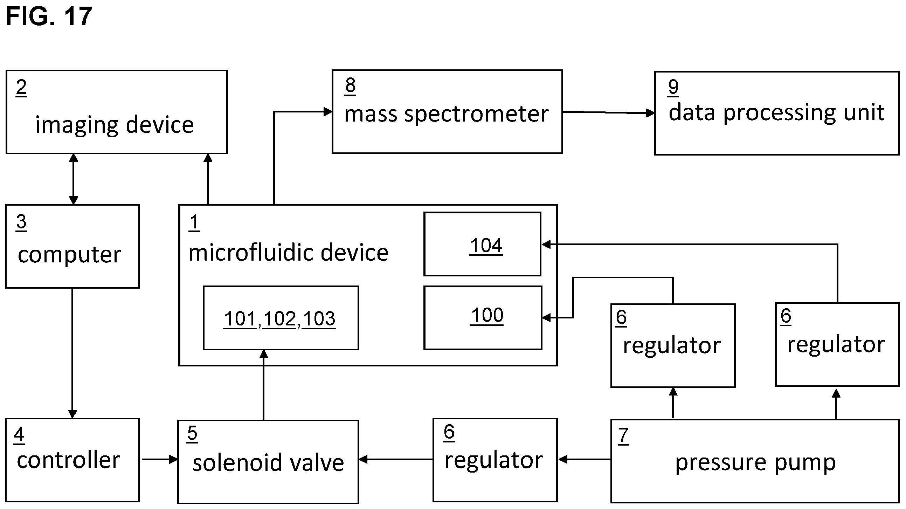

Fig. 17 shows a schematic overview of a system comprising a microfluidic device 1 according to a preferred embodiment of the present invention, where the microfluidic device 1 is used to process cells before analyzing their proteome using a mass spectrometer 8 and a data processing unit 9 connected to the mass spectrometer 8. The microfluidic device 1 is monitored by an imaging device 2, which is connected to a computer 3 for image acquisition. The computer 3 further communicates with a controller 4, the controller 4 controlling a solenoid valve 5. A pressure pump 7 is used control the pressure level of the fluids entering the microfluidic device 1 through the valve actuation inputs 101,102,103,104 or the inlet arrangement 100. A regulator 6 is placed between the pressure pump and the solenoid valve 5.

**LIST OF REFERENCE SIGNS**

| | | | |
|---|---|---|---|
| 1 | microfluidic device | 31 | outlet valve |
| 2 | imaging device | 32 | capillary |
| 3 | computer | 33 | fluid interface |
| 4 | controller | 40 | waste channel |
| 5 | solenoid valve | 41 | waste outlet |
| 6 | regulator | 42 | flushing channel |
| 7 | pressure pump | 50 | distribution channel |
| 8 | mass spectrometer | 60 | inlet channel |
| 9 | data processing unit | 61 | inlet valve |
| 10 | cell processing unit | 70 | sample fluid |
| 11 | main microchannel | 71 | cell (of a first cell type) |
| 12 | trapping region | 72 | additional sample fluid |
| 13 | input valve | 73 | cell (of a second cell type) |
| 14 | sieve valve | 80 | first reaction fluid |
| 15 | selection region | 81 | second reaction fluid |
| 16 | entrance valve | 90 | flushing fluid |
| 17 | rejection valve | 91 | control fluid |
| 18 | escape valve | 92 | control tube |
| 19 | escape channel | 92' | inlet tube |
| 20 | reaction unit | 93 | control channel (for inlet valves) |
| 21 | first pre-chamber | | |
| 211 | second pre-chamber | 94 | control channel (for entrance valves) |
| 22 | first connection valve | | |
| 221 | second connection valve | 95 | control channel (for processing valves) |
| 23 | first bypass valve | | |
| 231 | second bypass valve | 96 | control channel (for sieve valves) |
| 232 | flushing valve | | |
| 24 | first bypass channel | 100 | inlet arrangement |
| 241 | second bypass channel | 101 | inlet valve actuation input |
| 25 | lysis chamber | 102 | entrance valve actuation |
| 26 | digestion chamber | | input |
| 27 | separation valve | 103 | processing valve actuation |
| 28 | stirring valve | | input |
| 30 | outlet arrangement | 104 | sieve valve actuation input |
| 111 | corner area | | |
| 112 | indentation | | |
| 113 | contacting area | | |
| 201 | flow layer | | |
| 202 | control layer | | |
| 203 | substrate | | |

## Claims

1. A method for analyzing cells, the method comprising:
feeding a sample fluid (70) comprising a suspending medium and cells (71) suspended in the suspending medium to a microfluidic device (1) comprising at least one cell processing unit (10), each cell processing unit (10) comprising a main microchannel (11), the main microchannel (11) comprising a trapping region (12), a reaction unit (20) arranged downstream of the trapping region (12), and an outlet arrangement (30) arranged downstream of the reaction unit (20), the trapping region (12) being delimited by an arrangement of valves comprising:
an input valve (13) arranged at an upstream end of the trapping region (12), the input valve (13) being configured for blocking and unblocking a flow of the sample fluid (70) into the trapping region (12), and
a sieve valve (14), in particular a v-type valve, that connects the trapping region (12) to the reaction unit (20), the sieve valve (14) being able to assume a retaining state in which the sieve valve (14) is capable of retaining cells (71) while letting the suspending medium pass from the trapping region (12) into the reaction unit (20), and to assume an open state in which the sieve valve (14) allows cells (71) to pass from the trapping region (12) into the reaction unit (20);
establishing a flow of the sample fluid (70) through the trapping region (12) by opening the input valve (13) while the sieve valve (14) assumes said retaining state, whereby one or more cells are trapped in the trapping region (12);
interrupting the flow of the sample fluid (70) through the trapping region (12);
establishing a flow of a reaction fluid (80) through the trapping region (12) while the sieve valve (14) assumes said open state, such that the reaction fluid (80) transfers the trapped cells (71) from the trapping region (12) into the reaction unit (20);
decomposing the transferred cells (71) into cell fragments through a decomposition process;
opening an outlet valve (31) arranged between the reaction unit (20) and the outlet arrangement (30), whereby the cell fragments are collected in the outlet arrangement (30), and
analyzing the collected cell fragments.

2. The method of claim 1, further comprising
inducing cell-to-cell interactions between at least two cells trapped in the trapping region (12) by forcing the at least two cells into physical contact in a portion of the sieve valve (14) while the sieve valve (14) assumes its retaining state.

3. The method of claim 1 or 2,
wherein the first sample fluid (70) comprises cells of at least two different cell types and wherein the cells trapped in the trapping region (12) comprise cells of at least two different cell types; or
wherein the method further comprises:
providing at least one additional sample fluid (72), the at least one additional sample fluid (72) comprising cells (73) of a different cell type than the cell type of the cells (71) suspended in the first sample fluid (70),
interrupting the flow of the first sample fluid (70);
establishing a flow of the at least one additional sample fluid (72) through the trapping region (12), and
trapping, in the trapping region (12), at least one cell (73) that has a different cell type than a previously trapped cell (71) that is already retained in the sieve valve (14).

4. The method of any one of the preceding claims,
wherein the reaction unit (20) comprises a first pre-chamber (21), the first pre-chamber (21) being delimited by at least the sieve valve (14), a first connection valve (22) that connects the first pre-chamber (21) to a downstream portion of the reaction unit (20), and a first bypass valve (23) that connects the first pre-chamber (21) to a first bypass channel (24), the first bypass channel (24) optionally opening out into a waste channel (40), and
wherein the method further comprises:
establishing a flow of a flushing fluid (90) through the trapping region (12) while at least one cell (71) is retained by the sieve valve (14) in its retaining state, the flushing fluid (90) flowing from the trapping region (12) through the sieve valve (14) into the first pre-chamber (21) and exiting the first pre-chamber (21) through said first bypass valve (23).

5. The method of claim 2 or 3 in combination with claim 4,
wherein the downstream portion of the reaction unit (20) comprises a second pre-chamber (211) arranged downstream of the first pre-chamber (21), the second pre-chamber (211) being delimited by the first connection valve (22), the first connection valve (22) connecting the first pre-chamber (21) to the second pre-chamber (211), the second pre-chamber (211) being further delimited by a second connection valve (221) that connects the second pre-chamber (211) to a further part of the reaction unit (20) downstream of the second pre-chamber (211), a second bypass valve (231) that connects the second pre-chamber (211) to a second bypass channel (241), the second bypass channel (241) optionally opening out into the waste channel (40), and a flushing valve (232) that connects the second pre-chamber (211) to a flushing channel (42), and
wherein the method further comprises:
opening the sieve valve (14) and using the flow of the flushing fluid (90) to transfer the at least two cells that have been forced into physical interaction in a portion of the sieve valve (14) into the first pre-chamber (21);
opening the first connection valve (22) and using the flow of the flushing fluid (90) to transfer at least one of said at least two cells into the second pre-chamber (211), while at least one of the at least two cells remains in the first pre-chamber (21);
closing the first connection valve (22);
opening the first bypass valve (23) and using the flow of the flushing fluid (90) to transfer the at least one remaining cell from the first pre-chamber (21) into the first bypass channel (24), or
establishing a flow of the flushing fluid (90) through the flushing channel (42) into the second pre-chamber (211) by opening the flushing valve (232) and opening the second bypass valve (231), and using the flow of the flushing fluid (90) to transfer the at least one cell present in the second pre-chamber (211) from the first second pre-chamber (211) into the second bypass channel (241).

6. The method of any one of the preceding claims,
wherein the main microchannel (11) comprises a selection region (15) arranged upstream of the trapping region (12), the selection region (15) being delimited by at least an entrance valve (16), the input valve (13) and a rejection valve (17), the rejection valve (17) connecting the selection region (15) to a waste channel (40), and
wherein the method further comprises:
capturing at least one cell (71) in the selection region (15);
imaging the at least one captured cell (71) with an imaging device (2), in particular an optical microscope, and evaluating whether the at least one captured cell (71) fulfils a set of predetermined criteria;
opening the input valve (13) and using the flow of the sample fluid (70) to transfer the at least one cell (71) into the trapping region (12) if the at least one cell (71) fulfils the set of predetermined criteria; or
opening the rejection valve (17) and using the flow of the sample fluid (70) to transfer the at least one cell (71) into the waste channel (40) if the at least one cell (71) does not fulfil the set of predetermined criteria.

7. The method of any one of the preceding claims,
wherein the arrangement of valves delimiting the trapping region (12) further comprises an escape valve (18), the escape valve (18) connecting the trapping region (12) to an escape channel (19), the escape channel optionally opening out into a waste channel (40).

8. The method of any one of the preceding claims,
wherein decomposing the cells comprises a lysis step for extracting the cell proteome and a digestion step for fragmenting the proteome into peptides, and
wherein analyzing the cell fragments comprises a proteomic analysis step using mass spectrometry,
wherein the reaction unit (20) preferably comprises a lysis chamber (25) and a digestion chamber (26), the digestion chamber (26) being separated from the lysis chamber (25) by a separation valve (27), and
wherein the method further comprises:
performing the lysis step in the lysis chamber (25) using a first reaction fluid (80) while the separation valve (27) assumes a closed state;
establishing a flow of a second reaction fluid (81) through the lysis chamber (25) into the digestion chamber (26) to transfer the lysed cells from the lysis chamber (25) through the separation valve (27) into the digestion chamber (26) while the separation valve (27) assumes an open state;
performing the digestion step using the second reaction fluid (81) in the digestion chamber (26); and
optionally, periodically opening and closing a stirring valve (28) arranged in the digestion chamber (26) in order to accelerate the digestion step.

9. The method of any one of the preceding claims,
wherein the microfluidic device (1) comprises at least two cell processing units (10) that are arranged in parallel, said at least two cell processing units (10) being connected by a distribution channel (50) upstream of said at least two cell processing units (10), and
wherein at least two inlet channels (60) are arranged upstream of the distribution channel (50), each inlet channel (60) being connected to the distribution channel (50) by an inlet valve (61);
the method comprising:
establishing a flow of a first sample fluid (70) comprising cells of a first cell type through at least one inlet channel (60) into the distribution channel (50);
causing at least one first selected cell processing unit (10) to receive said first sample fluid (70) from the distribution channel (50);
trapping cells of the first cell type in the at least one first selected cell processing unit (10);
interrupting the flow of the first sample fluid (70);
establishing a flow of at least one additional sample fluid (72) comprising cells of at least one additional cell type through at least one inlet channel (60) into the distribution channel (50);
causing at least one second selected cell processing unit (10) to receive said at least one additional sample fluid (72), the at least one second selected cell processing unit (10) being identical to the at least one first selected cell processing unit (10) or different therefrom;
trapping cells of the at least one additional cell type in the at least one second selected cell processing unit (10);
interrupting the flow of the at least one additional sample fluid (72);
establishing a flow of at least one reaction fluid (80) through at least one inlet channel (60) into the distribution channel (50); and
causing at least one third selected cell processing unit (10) to receive said at least one reaction fluid (80), the at least one third selected cell processing unit (10) comprising the at least one first and/or second selected cell processing units.

10. A microfluidic device (1) comprising at least one cell processing unit (10), the at least one cell processing unit (10) comprising a main microchannel (11), the main microchannel (11) being capable of channelling a flow of a sample fluid (70) comprising a suspending medium and cells (71) suspended in the suspending medium, the main microchannel (11) comprising a trapping region (12), a reaction unit (20) arranged downstream of the trapping region (12), and an outlet arrangement (30) arranged downstream of the reaction unit (20), the trapping region (12) being delimited by an arrangement of valves comprising:
an input valve (13) arranged at an upstream end of the trapping region (12), the input valve (13) being configured for blocking and unblocking the flow of the sample fluid (70) into the trapping region (12), and
a sieve valve (14), in particular a v-type valve, that connects the trapping region (12) to the reaction unit (20), the sieve valve (14) being able to assume a retaining state in which the sieve valve (14) is capable of retaining cells (71) while letting the suspending medium pass from the trapping region (12) into the reaction unit (20), and to assume an open state in which the sieve valve (14) allows cells (71) to pass from the trapping region (12) into the reaction unit (20).

11. The microfluidic device of claim 10,
wherein the main microchannel (11) comprises a selection region (15) arranged upstream of the trapping region (12), the selection region (15) being delimited by at least an entrance valve (16), the input valve (13) of the trapping region (12), and a rejection valve (17), the rejection valve (17) connecting the selection region (15) to a waste channel (40).

12. The microfluidic device of claim 10 or 11,
wherein the arrangement of valves delimiting the trapping region (12) further comprises an escape valve (18), the escape valve (18) connecting the trapping region (12) to an escape channel (19).

13. The microfluidic device of any one of claims 10-12,
wherein the reaction unit (20) comprises a first pre-chamber (21), the first pre-chamber (21) being delimited by at least the sieve valve (14), a first connection valve (22) that connects the first pre-chamber (21) to a downstream portion of the reaction unit (20), a first bypass valve (23) that connects the first pre-chamber (21) to a first bypass channel (24), the first bypass channel (24) optionally opening out into a waste channel (40), and
wherein the downstream portion of the reaction unit (20) comprises a second pre-chamber (211) arranged downstream of the first pre-chamber (21), the second pre-chamber (211) being delimited by the first connection valve (22), the first connection valve (22) connecting the first pre-chamber (21) to the second pre-chamber (211), the second pre-chamber (211) being further delimited by a second connection valve (221) that connects the second pre-chamber (211) to a further part of the reaction unit (20) downstream of the second pre-chamber (211), a second bypass valve (231) that connects the second pre-chamber (211) to a second bypass channel (241), the second bypass channel (241) optionally opening out into the waste channel (40), and a flushing valve (232) that connects the second pre-chamber (211) to a flushing channel (42).

14. The microfluidic device of any one of claims 10-13,
wherein the reaction unit (20) comprises a lysis chamber (25) and a digestion chamber (26) arranged downstream of the lysis chamber (25), the digestion chamber (26) being separated from the lysis chamber (25) by at least one separation valve (27), and
wherein a stirring valve (28) is arranged in the digestion chamber (26).

15. The microfluidic device of any one of claims 10-14,
wherein the microfluidic device (1) comprises at least two cell processing units (10) that are arranged in parallel, the at least two cell processing units (10) being connected by a distribution channel (50) arranged upstream of the at least two cell processing units (10), and
wherein at least two inlet channels (60) are arranged upstream of the distribution channel (50), each inlet channel (60) being connected to the distribution channel (50) by an inlet valve (61).
